# EUROPEAN PATENT SPECIFICATION

(11) **EP 1 684 815 B1**
(45) Date of publication and mention of the grant of the patent: **17.12.2008**
(21) Application number: 04790224.2
(22) Date of filing: 08.10.2004
(51) Int. Cl.: A61L 27/02, A61L 27/04, C12N 5/06

(54) **CARTILAGE REGENERATION BY GENERATION OF CHONDRONS UNDER HIGH CONCENTRATIONS OF MAGNESIUM**
KNORPELREGENERATION MIT GENERATION VON CHONDRONEN UNTER HOHER KONZENTRATION VON MAGNESIUM
REGENERATION DE CARTILAGES AVEC GENERATION DE CHONDRONS EN PRESENCE D'UNE CONCENTRATION ELEVEE DE MAGNESIUM

(30) Priority: 10.10.2003 EP 03022780; 10.10.2003 US 509942 P
(43) Date of publication of application: 02.08.2006
(73) Proprietor: KW2 Implantattechnologie GmbH, 30171 Hannover (DE)
(72) Inventor: WITTE, Frank, 30625 Hannover (DE); WINDHAGEN, Henning, 30159 Hannover (DE); KAESE, Volker, 30161 Hannover (DE); FEYERABEND, Frank, 22529 Hamburg (DE)
(74) Representative: Kröncke, Rolf
(86) International application number: PCT/EP2004/011287
(87) International publication number: WO 2005/039662

(56) References cited:
- EP-A- 0 339 607
- WO-A-01/82994
- WO-A-97/18299
- WO-A-02/061052
- WO-A-02/094377
- US-A- 4 954 349
- US-A- 5 206 023
- US-A1- 2002 082 623
- US-A1- 2003 103 947
- US-B1- 6 211 143
- US-B1- 6 248 368

## Description

The present invention relates to a method for the generation of chondrons and of cartilaginous tissue. In particular, the present invention relates to the ability of Mg ions to stimulate the growth and regeneration of chondrons, particularly of pseudo-chondrons as an intermediate in the regeneration and growth of cartilaginous tissue. Especially, unphysiologically high extracellular concentrations of Mg are able to regenerate hyaline cartilage, elastic cartilage and/or fibrocartilage via the intermediate form of chondrons.

### Background art

Articular cartilage has a limited ability for the repair of joint surface damages. To date, there is no in vivo or in vitro treatment that fully restores the cartilage damages (Hunziker, E.B. 2002, Osteoarthiritis.Cartilage 10, 432-463). However, a suitable concept for the treatment of joint damages is based on cartilage produced in vitro. Presently used processes/methods for the production of artificial cartilage are in vitro culturing systems based on the alginate system.

The alginate system is based on alginate, a linear copolymer of β-D-mannuronic acid and α-L-glucuronic acid, which is harvested from brown algae. It has the ability to polymerize to a gel when bringing into contact with calcium-ions. This process is reversible, the de-polymerization is induced by citrate. Based on these properties it is possible to culture cells in and to recover them from the alginate for further cultivation. The main advantage of this system for chondrocytes is that the cells can be re-differentiated by minimizing the cell-cell contacts, which are a signal for chondrogenesis (Bonaventure J, et al. 1994, Exp Cell Res 212(1):97-104.).

However, especially the use of growth factors and/or cytokines make the process/method of generating cartilage in vitro very costly. Furthermore, the long term safety of these additions is not proved.

Different types of cartilages are determined by chondrocytes and their extracellular matrix (ECM). These different types of cartilage provide different anatomical and functional properties according to their histological morphology. In particular, chondrocytes, the main cellular component of the cartilage, are slow growing cells that secret extracellular matrix proteins to form the different types of cartilages found in the body. Depending on the structure of the cartilage, the cartilaginous tissue appears histologically as hyaline, elastic or fibrocartilage. A combination of these tissues in one functional unit is possible.

Hyaline cartilage represents the most common type of cartilage in the body and contains characteristally collagen type II fibres in its ECM. Typically, hyaline cartilage can be found in articular joints, costal cartilage (ribs), nose, larynx, and growth plate. Another type of cartilage is the elastic cartilage. This kind of cartilage may be found in ear, trachea and larynx, i.e. the epiglottis. The third type of cartilaginous tissue is present in symphysis, intervertebral disci, parts of the articular joints, menisci and in other joints, like the jaw-joint.

Of course, it is possible to find a combination or intermediates of these types of cartilage, for example, the epiphyseal cartilage in the growth or cartilage plate.

In many diseases and disorders a damage of the cartilage occurs. However, regardless of their etiology, cartilage defects, e.g. of articular joints, and their treatment remain one of the unsolved problems in medicine.

As stated above, the most promising method for the treatment of cartilage regeneration, e.g. of hyaline cartilage, is the harvesting of patient cells followed by in vitro cultivation of these cells to chondrocyte-construct in an alginate system and reimplantation of this construct into the cartilage defect of the same patient.

A good cartilage construct is determined by chondrons arranged in a functional order in the cartilage construct which are derived from chondrocytes.

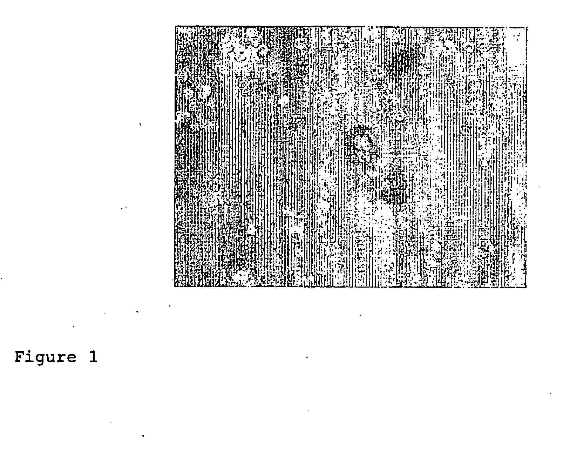

Chondrocytes are derived from mesenchymal cells that have a characteristic phenotype based primarily on the type of extracellular matrix they produce. The precursor chondrocytes produce type I collagen but when they become committed to chondrocytes present in the various types of cartilage, they synthesize type II collagen. In addition said chondrocytes produce proteoglycan aggregate which has glycosaminoglycans that are highly sulphated. This state of the chondrocytes which resembles the appearance of chondrocytes in native cartilage is also sometimes referred to as chondrons. Chondrons consist of at least one chondrocyte, which is/are encapsulated in extracellular matrix, consisting of at least collagen type II, (type IV and various matrix proteins) and GAGs. Depending on the type of cartilage, the chondrons consist of 1 to 3 chondrocytes in case of elastic cartilage or of at least 2 chondrocytes in case of hyaline cartilage. In particular, chondrons are characterized in expressing type II collagen and glucosaminoglycans (GAG). Moreover, chondrons are known to express additional cartilage specific proteins and genes such as collagen type IX, XI, aggrecan, chondroitin sulphate, keratin sulphate and SOX9. In the literature, the term pseudo-chondrons is sometimes used. Pseudo-chondrons (see for example Fig.1) designate chondrons which have not been formed in their natural environment but e.g. have been build up in vitro or maintained ex vivo.

A definition and a review of chondrons can for example be found in Bonaventure, J., et al. 1994, Exp. Cell Res. 212, 97-104, which is herewith incorporated by reference.

In case of cartilage diseases of articular joints, there are two major diseases that affect cartilage, namely osteoarthritis and rheumatoid arthritis; both osteoarthritis and rheumatoid arthritis result in degradation and degeneration of the articular cartilage. Osteoarthritis is primarily a non-inflammatory disorder of movable joints characterized by an imbalance between the synthesis and degradation of the articular cartilage, leading to the classic pathologic changes of wearing away and destruction of cartilage.

Rheumatoid arthritis is an autoimmune systemic disease accompanied by severe inflammation of the joints. In most patients rheumatoid arthritis begins with a general feeling of malaise, fatigue, often accompanied by diffuse musculoskeletal pain. Eventually the disease progresses resulting in pain on motion, tenderness, swelling and deformation of multiple joints; because rheumatoid arthritis is a systemic disease, it may be accompanied by extra-articular complications, such as anemia, vasculitis, scleritis, pleurisy, pericarditis, and peripheral neuritis.

Further, damage of cartilage and/or the underlying bone occurs post-traumatically or in orthopaedic surgery. In these cases regeneration or replacement of the destroyed cartilaginous tissue is necessary.

Moreover for the re-building of cartilage, e.g. cartilage present in ear, nose, intervertebral disci or menisci, it is necessary to engineer new cartilage or precursor cell material for the development of cartilage, like chondrons, in vitro and, subsequently, transplant the in vitro generated chondrons or cartilaginous tissue into the patient. However, re-building or tissue engineering of cartilage tissue presently requires the use of a scaffold, cells, preferably obtained from the patient to be treated, and a cocktail of various growth and differentiation factors as well as cytokines and/or hormones. In particular the necessity of using a cocktail of various growth and differentiation factors renders this method expensive.

### Summary of the present invention

In view of the above described problems, the object of the present invention is to improve the growth and regeneration of cartilaginous tissue.

In particular, the invention relates to an in vitro method for the generation of chondrons and of cartilaginous tissue including the step of cultivating chondrocytes which may be derived from precursor cells in unphysiologically high extracellular concentrations of Mg. The cultivation step at extracellular unphysiologically high concentrations of Mg comprises the step of increasing at least once the concentration of Mg ions in culture.

Especially, the present invention relates to the generation of chondrons and cartilaginous tissue in vitro.

These in vitro generated chondrons also called pseudo-chondrons and the cartilaginous tissue obtained therefrom can be used in the treatment of cartilaginous tissue after orthopaedic surgery or posttraumatic and/or degenerative damage of the cartilage.

In particular, the method is characterized in comprising the step of increasing the extracellular unphysiologically high concentration of Mg at least once during cultivation.

Thus, the invention describes *inter alia* methods for the generation of cartilaginous tissue via chondrons as an intermediate. Said tissue or chondrons which are generated in vitro may be used for the treatment of rheumatoid arthritis and osteoarthritis and post-traumatic changes and other conditions that manifest cartilage degradation of the joints, and also includes the destructive diseases of other cartilages, such as degenerative changes and/or post-traumatic changes in the vertebral disci and/or menisci and/or in the cartilage of the nose and/or of the ear.

### Short description of the figures

Figure 1: Pseudo-chondrons after recovery from the alginate. Human chondrocytes were incubated for 21 days in alginate. After elution they were stained with 1,9-dimethylmethyleneblue-chloride (DMMB). Chondrocytes build a pseudo-chondron and secreted matrix. Phase contrast microscopy, 100fold magnification.
Figure 2: Cell Counts with different concentrations of magnesium in the culture system.
Figure 3: Matrix- and collagen- contents after the differentiation stage. Left: glycosaminoglycan (GAG) content, Right: Western Blot of collagen type II after differentiation stage. Lanes: 1: Marker for Collagen Type I (50ng); 2: Marker for collagen type II (50ng); 3: Control (no magnesium); 4: 5mM MgSO4-solution; 5: 10mM MgSO4-solution; 6: 20mM MgSO4-solution.
Figure 4: Matrix-synthesis with different supplementations during stage 2 (first character) and aggregation culture (second character). K = 0mM magnesium (Control); IT = IGF-I + TGF-β1 + IL-4; x Mg = mMol MgS04
Figure 5: Matrix synthesis with different supplementations during stage 1 (first character), stage 2 (second character) and aggregation culture (third character). Abbreviations: K = 0mM magnesium (Control); Mg = 10mM MgSO4; F = bFGF; ITIL = IGF-I + TGF-β1 + IL-4

### Detailed description of the invention

It is noted that as used herein the following terms have the meaning as indicated below.

The term "cultivation of cells" as used herein is intended to mean that the cells are kept under conditions allowing the cells to growth and/or to differentiate. Cultivation of cells can be effected in vivo or in vitro. Thus, the claimed method comprising the step of cultivation of cells at unphysiologically high extracellular concentrations of Mg can be, conducted in vivo, e.g. in natural environment, in mammals other than humans or in vitro.

The term "generation" as used herein is intended to mean the differentiation as well as the proliferation of cells or precursor cells.

The term "unphysiologically high extracellular concentration of Mg" is intended to mean that the concentration of the Mg ion in the culture is above the physiological level normally present in the body the cells are derived from. For example, in humans the extracellular concentration of Mg is about 0,9 mMol. Thus, unphysiologically high concentration means that the concentration of Mg in the extracellular compartment is above said concentration.

The term "at least once the Mg concentration is increased" is intended to mean that during in vivo or in vitro cultivation the already unphysiologically high concentration of Mg is further increased by adding additional Mg to the culture. The addition may be effected in vivo by administrating an agent which results in increasing the Mg concentration in the extracellular compartment or in vitro by adding additional Mg, e.g. in form of salts, to the culture system.

The term "agent" means a pharmaceutical or medicinal composition containing as an active ingredient Mg or Mg-derivatives as defined herein. In particular, the agent may be in form of a gel, paste, tablet, injection or infusion to be applied locally.

The term "cartilaginous tissue" or "cartilage" as used herein means any type of cartilage or tissue comprising cartilage-like structures. In particular, the above terms encompass the hyaline-, elastic-, and fibrocartilage and intermediates or mixed structures thereof.

The term "Mg or Mg-derivative" as used herein is intended to mean magnesium ions in the free form or as the salts including complexed forms.

The magnesium cation is an essential mineral for many animals, including mammals, and especially for humans. As such, magnesium is also a cofactor in numerous enzymatic reactions. It is involved in phosphate transfer from ADP and ATP muscle contractility, integrin activation and neuronal transmission. The majority of magnesium in the human body is located in the bones in the form of phosphates and carbonates, and the remainder is found principally in the liver and muscles; red blood cells also contain magnesium. Magnesium inhibits nerve impulses and relaxes muscle contractions, thereby functioning antagonistically to calcium. On the other hand, like calcium, magnesium can bind phosphates and can substitute for calcium as a bone or tooth mineral.

Thus, only about 1% of the total magnesium present in the body is in the extracellular, liquid compartment, mainly in the serum. The magnesium concentration in the serum is typically about 1,8 to 2,2 mg/dl; corresponding to about 0,9 mmol/1.

In the blood serum, magnesium can be found mainly in three different forms, i.e. protein-bound magnesium, complexed magnesium or magnesium ions.

The distribution of magnesium varies with age and within different species. That means e.g. the concentration of magnesium in bones and menisci decrease with age.

Various magnesium compounds have been used via intramuscular, oral, and intravenous routes of administration. For example, magnesium acetate is used as a source of magnesium and as an acetate supply of bicarbonate in hemodialysis or peritoneal dialysis solutions; magnesium chloride is likewise used in dialysis solutions.

The inventors now found that the cultivation of chondrocytes or precursor cells thereof at unphysiologically high extracellular concentrations of magnesium allows for the generation of chondrons, an intermediate in the tissue regeneration of cartilaginous tissue. In particular, further increasing the extracellular magnesium concentration above physiologic level at least once during cultivation leads to chondrons which may be further differentiated to cartilaginous tissue.

Thus, the regulated elevation of magnesium concentration above physiologic level including at least once the step of further increasing the Mg concentration in the extracellular compartment by applying magnesium in form of e.g. an agent accelerates cartilage growth and/or regeneration in mammals.

Preferably, the magnesium concentration is initially at least three times above the physiologic level, more preferably five times above the physiologic level of the respective compartment, i.e. the extracellular compartment of the cartilage or, when used in tissue engineering, of the cell culture medium. Preferably, the Mg concentration is in the range of from 11 to 25 mMol.

After increasing the magnesium concentration at least once during cultivation, the magnesium concentration is in the range of from 21 to 65 mMol.

The Mg concentration may be later decreased after having once increased the same. That means, after increasing the Mg concentrations once during cell cultivation and forming of chondrons, the Mg concentration may be decreased at e.g. physiological levels or even below physiological extracellular levels.

In another aspect of the invention it is provided a method of using magnesium or magnesium-derivatives as defined herein to promote specific stages of chondrocyte and/or cartilage maturation. Thus, by time-controlled application it is possible to regulate osteogenesis and chondrogenesis of the regenerating bone and cartilaginous tissue in order to optimise the newly formed structure of the cartilage.

In a further embodiment, the substrate or agent containing Mg or Mg derivatives allows for differently regulated release of predetermined amounts of Mg into the environment. Thus, it is possible to promote or suppress specific stages of cartilage development.

In addition, the present invention allows to control the growth and development of artifical cartilage, e.g. for the use as a framework for organs. These artifical organs may be used in the replacement of the outer ear, valvular, nose or intervertebral discs or for the replacement of menisci or articular joints.

In another embodiment, Mg or Mg-derivatives are used in in vitro tissue engineering of cartilaginous tissue. Methods for the generation of cartilaginous tissue via tissue engineering are known in the art.

However, the methods presently described in the art requires the use of expensive cocktails of various growth factors or differentiating factors along with the respective chondrocytes or chondroblasts, bone-marrow stromal cells, synovial cells and various precursor cells. The use of Mg or Mg derivatives as defined herein allows for the reduction of other growth factors thus reducing the costs and, additionally, allows for a controlled generation and optimized development of the cartilage. However, the cultivation may take place in the presence of at least one growth factor, cytokine and/or hormone. In addition, foetal calf serum or mammalian serum like human serum may be present.

The magnesium compound added to the culture is preferably magnesium sulphate or magnesium chloride, but not limited to these compounds.

When in vitro cultivation for tissue engineering of cartilaginous tissue is performed, the first part of the cultivation is conducted in tissue culture systems as a monolayer culture, preferably in a medium supplemented with FCS. During the first stage of cultivation, the magnesium concentration is in the range of from 11 to 25 mMol.

In the second stage of cultivation, i.e. when the magnesium concentration is increased once in comparison to the first stage, the cultivation is preferably performed as a cultivation of the cells embedded in alginate and cultured in medium supplemented with serum from a mammal. During this second stage, the magnesium concentration is in the range of from 21 to 65 mMol.

The cells to be cultivated may be chondrocytes or chondrocytes differentiated from chondrocyte precursor cells and/or from mesenchymal stem cells and/or embryonic stem cells and/or adult stem cells. Preferably, the cells are of human origin.

In addition, Mg and Mg-derivatives can be used in a method for the preparation of cartilaginous tissue in gene therapy.

Also encompassed is the use of said agents for treating patients suffering on cartilage diseases, disorders or damages due to surgery, trauma, degeneration or as a consequence of other types of diseases. The Mg or Mg-derivatives may be administered into or in the vicinity of the cartilage to be treated, thus, increasing the Mg concentration in the extracellular compartment to unphysiologically high concentrations. The administration may be in form of pharmaceutical compositions like infusions, injections or via catheter. Alternatively, a substrate may be used which allows for the release of Mg or Mg derivatives by bio- and/or chemical and/or physical degradation. When treating patients it is necessary to elevate the level of magnesium in the extracellular compartment of the cartilage above physiologic level, preferably at least 300% above said level.

Further increasing the Mg concentration may be achieved by administering appropriate agents or pharmaceutical compositions.

Thus, the present invention relates to the use of the agents and the chondrons and cartilaginous tissue for treating or preventing cartilage diseases, disorders or damages characterized in administering magnesium into or in the vicinity of the cartilage. Further, the present invention relates to said use in methodes for growing or regenerating cartilaginous tissue characterized in elevating the magnesium concentration in the cartilaginous tissue above physiologic level whereby said magnesium concentration is further increased at least once during cultivation. In particular, the method may comprise administering the magnesium in form of a substrate or an agent.

The local administration of Mg or Mg-derivatives as defined herein allows for an optimised regeneration and/or growth of cartilaginous tissue. In particular by timely limited administration, e.g. by using degradable substrate containing Mg or Mg-derivatives, it is possible to accelerate the tissue regeneration in chondral and/or in osteochondral defects.

Thus, Mg and Mg-derivatives can positively influence cartilage formation in vivo and in vitro.

Of course, it is possible to combine the Mg or Mg-derivatives being present in form of a substrate or agent with at least one further compound known in the art to promote the growth and/or regeneration of cartilaginous tissue or which is used in the prophylaxis or treatment of cartilage diseases, disorders or damages.

The formulation of suitable substrates and agents in form of pharmaceutical composition is known to the skilled person.

Moreover, the dosage of the Mg or Mg-derivatives administered may vary depending on the conditions of the individual, age, body weight, etc. The skilled person knows how to provide Mg or Mg-derivatives in an amount elevating the level of Mg above the physiologic level of the extracellular compartment of the cartilage or of the culture medium in case of tissue engineering.

Thus, the present invention may be used for the prophylaxis or treatment of chondral or osteochondral defects or damages. Further, the present invention is useful in treating the rupture or degeneration of meniscus or discus, like slipped discus. Moreover, the present invention relates to the use of magnesium or magnesium derivatives in degenerative, autoimmune or inflammatory diseases or trauma leading to hyaline, elastic and/or fibroelastic cartilage damage. Further, diseases causing growth disorders or growth disorders itself, which may affect directly or indirectly the cartilaginous tissue of the growth plate are encompassed in the present invention.

The method or use according to the present invention is applicable to mammals, i.e. humans and animals.

Alternatively, magnesium or magnesium derivatives as defined herein are useful for the preparation of artificial meniscus or discus to be used in meniscus or discus replacement, respectively. Moreover, the present invention is useful in ligament surgery. Cartilaginous tissue can e.g. be found on the insertion of the ligament. Thus, ligament surgery may encompass the use of magnesium or magnesium derivatives for promoting the generation of cartilaginous tissue being connected with the ligament.

### Examples

The following Examples illustrate the effects when using Mg or Mg-derivatives in a regulated manner on the regeneration of cartilage. However, the invention is not limited to or by the examples.

### 1. Proliferation stage

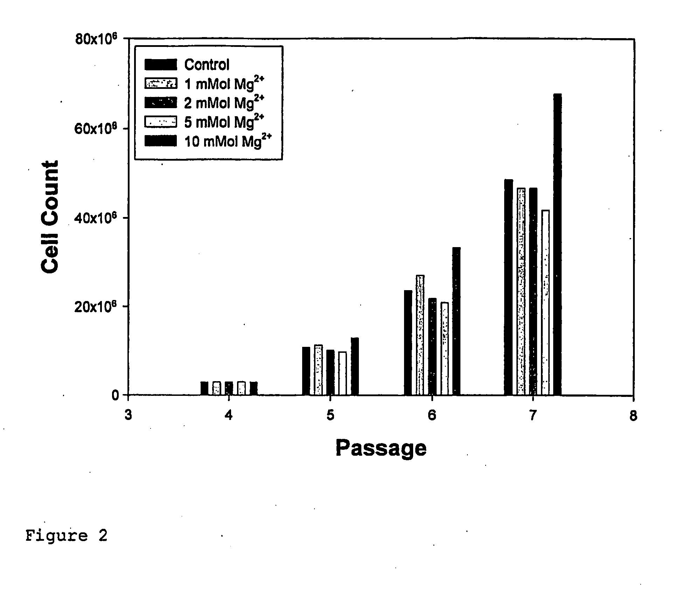

Human chondrocytes were seeded in an initial cell number of 5 x 10⁶ cells in cell culture flasks and cultivated in DMEM high glucose + 10 % by vol. of FCS + different concentrations of magnesium sulphate-solution (0, 1, 2, 5 and 10 mM). Medium was changed twice a week. Cells were passaged once per week with 0.25% by weight trypsin/EDTA, counted and reseeded (see Fig. 2). The cell count was determined with a CASY-cell-counter, Schärfe-System GmbH, Germany.

### 2. Differentiation stage

Human chondrocytes were proliferated in DMEM high glucose + 10% by vol. FCS + 10 ng/ml bFGF for six passages. After trypsination a washing step in HEPES-buffered saline (HBS, 0.15M NaCl / 25mM HEPES, pH 7.4) was performed. The cells were suspended in 1.2% by weight alginate in HBS in a density of about 1x10⁶ cells/ml. Cell suspension was introduced drop wise into glass culture flasks containing 0.1M CaCl2/25mM HEPES. An immediate polymerization of the alginate took place. The alginate beads were washed with HBS after 30 min.

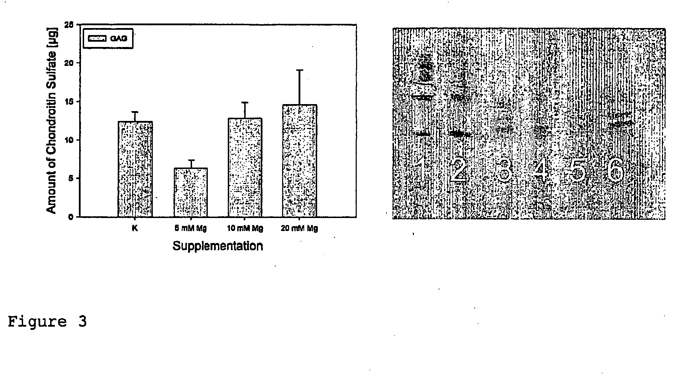

Afterwards the encapsulated cells were supplemented with DMEM high glucose + 10% by vol. human serum + 0.28mM ascorbic acid 2-phosphate + 1mM cysteine + different concentrations of magnesium sulphate solution (0, 5, 10 and 20mM) and cultivated for 23 days at 37°C, 5% CO2, 8% 02 and 95% atmospheric humidity with the medium changed every two days. Afterwards the cells were isolated by dissolving the alginate with dissolving buffer (0.15mM NaCl + 55mM tri- sodium citrate + 25mM HEPES) at 37°C in a shaking water bath for 20 to 25 min. An analysis of the amount of chondroitin sulphate and the collagen II content is shown in Figure 3.

### 3. Chondrogenesis after magnesium supplementation in stage 1 and/or 2

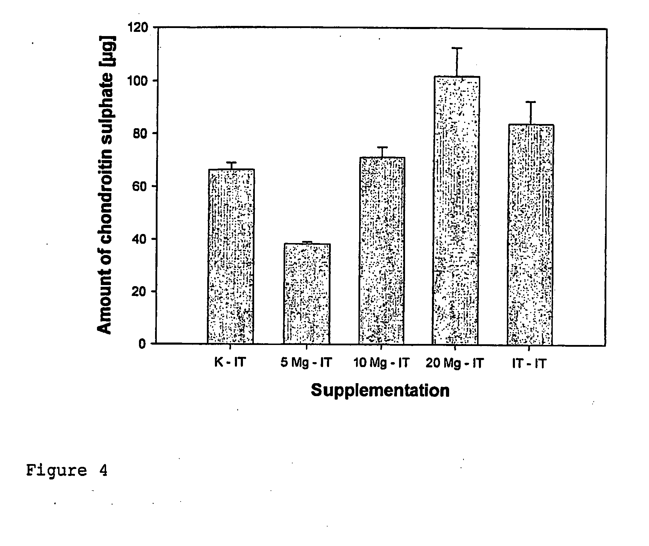

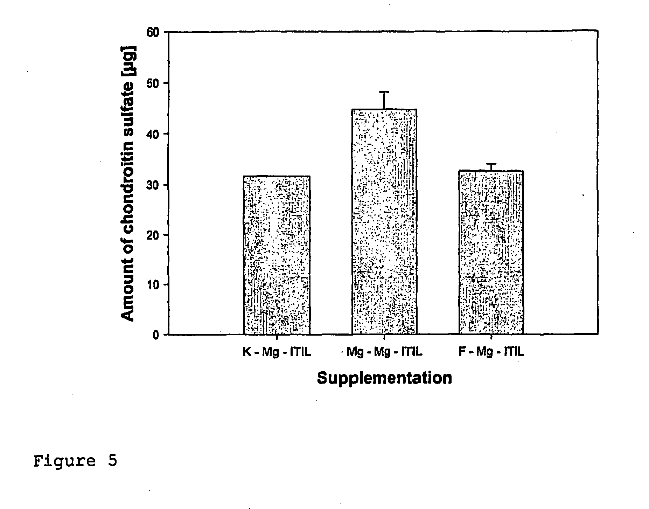

a) Chondrocytes were treated as in example 2. After the recovery out of the alginate they were centrifuged to aggregation pellets of 5x10⁵ cells. These were cultivated in DMEM high glucose + 10% by weight human serum + 0.28mM ascorbic acid 2-phosphate + 1mM cysteine + 100ng/ml insulin-like growth factor I + 20ng/ml transforming growth factor β1 and 10ng/ml Interleukin-4 for three weeks at 37 °C, 5%CO2, 19% 02 and 95% atmospheric humidity with the medium changed every two days. Culture was performed in agarose-coated (1% by weight agarose in a. dest.) 12-well dishes with 3ml of medium (see Fig. 4).
b) Chondrocytes were proliferated as in example 1 with no magnesium or 5mM magnesium or bFGF. They were differentiated as in example 2 with 10mM magnesium. Afterwards they were cultivated as in example 3 a). See Fig. 5.

The determination of the amount of chondroitin sulphate was performed by known method with the help of 1,9-dimethylmethylenblue-chloride and measured via fluorometric measurement at an extinction of 530nm.

## Claims

1. Method for the *in vitro* generation of chondrons comprising the step of:
cultivation of cells in a medium having unphysiologically high extra cellular concentrations of magnesium (Mg),
**characterized in that** at least once the unphysiologically high extra cellular Mg concentration is increased during cell cultivation.

2. The method according to claim 1, wherein said magnesium is a solution of magnesium sulphate or magnesium chloride.

3. The method according to any one of claims 1 or 2, wherein said extra cellular concentrations of said magnesium solution range from about 12 mmol to about 65 mMol.

4. The method according to any one of the preceding claims, wherein the cultivation of the cells is further affected in the presence of foetal calf serum (FCS) or mammalian serum.

5. The method according to any one of the preceding claims, wherein the cultivation of the cells is further affected in the presence of at least one growth factor and/or cytokine and/or hormone.

6. The method according to any one of the preceding claims, wherein chondrocytes isolated from tissue of a mammal are cultivated.

7. The method according to any one of the preceding claims, wherein chondrocytes differentiated from chondrocyte precursor cells and/or from mesenchymal stem cells and/or embryonic stem cells and/or adult stem cells are cultivated.

8. The method according to claim 6, wherein the chondrocytes are of human origin.

9. The method according to any one of the preceding claims, wherein the cells, preferably chondrocytes, are seeded into tissue culture flasks and are cultivated in monolayer culture with medium supplemented with FCS and concentration of magnesium is initially in the range of 11 to 25 mMol.

10. The method according to any one of the preceding claims wherein when increasing the Mg concentration the cells are embedded in alginate and cultured in medium supplemented with serum from said mammal, the concentration of magnesium is increased to a range of 21 to 65 mMol.

11. The method according to claim 10 wherein the cultivation is effected under an oxygen partial pressure of 8 %.

12. A method for the preparation of cartilaginous tissue comprising the method according to any one of claims 1 to 11.

13. Use of the chondrons obtained according to any one of claims 1 to 11 for the preparation of cartilaginous tissue.

14. Cartilaginous tissue obtained according to a method of claim 12.

15. The use of Mg and Mg-derivatives and chondrons generated in vitro by a method according to any one of claims 1 to 11 for the preparations of a medicament for treating patients suffering on cartilage diseases, disorders or damages due to surgery, trauma, degeneration or as a consequence of other types of diseases whereby the extracellular magnesium concentration is increased at an unphysiological high extracellular level and said unphysiologically high extracellular Mg concentration is at least once increased further during treatment.

16. The use according to claim 15, wherein said magnesium is a solution of magnesium sulphate or magnesium chloride.

17. The use according to any one of claims 15 to 16, wherein the medicament further contains at least one growth factor and/or cited cytokine and/or hormone.

18. The use according to any one of claims 15 to 17, wherein the magnesium is adapted to the administered in form of a substrate or an agent.

19. The use according to any one of claims 15 to 18, wherein the medicament is adapted to be administered into or in the vinicity of the cartilage to be treated.

20. The use according to any one of claims 15 to 19, wherein the cartilage disease is any one of chondral or osteochondral defects or damages, the rupture or degeneration of meniscus or discus, in degenerative, autoimmunie or inflammatory disease or trauma leading to hyaline, elastic and/or fibroelastic cartilage damage.

21. The use of chondrons obtained according to any one of claims 1 to 11 or cartilaginous tissue according to claim 14 for the preparation of a medicament for treating patients suffering on cartilage diseases, disorders or damages due to surgery, trauma, degeneration or as a consequence of other types of diseases whereby the extracellular magnesium concentration is increased at an unphysiological high extracellular level and said unphysiologically high extracellular Mg concentratin is at least once increased further during treatment.

## Patentansprüche

1. Verfahren zur in-vitro-Generierung von Chondronen, umfassend den Schritt des:
Kultivierens von Zellen in einem Medium, das eine unphysiologisch hohe extrazelluläre Konzentration von Magnesium (Mg) aufweist,
**dadurch gekennzeichnet, dass** mindestens einmal die unphysiologisch hohe Magnesiumkonzentration während der Kultivierung der Zellen erhöht wird.

2. Verfahren nach Anspruch 1, wobei das Magnesium eine Lösung von Magnesiumsulfat oder Magnesiumchlorid ist.

3. Verfahren nach einem der Ansprüche 1 oder 2, wobei die extrazellulären Konzentrationen der Magnesiumlösung im Bereich von ca. 12 mMol bis 65 mMol liegt.

4. Verfahren nach einem der vorherigen Ansprüche, wobei die Kultivierung der Zellen weiterhin in Anwesenheit von fötalem Kälberserum (FCS) oder Säugetierserum durchgeführt wird.

5. Verfahren nach einem der vorherigen Ansprüche, wobei die Kultivierung der Zellen weiterhin in Anwesenheit von mindestens einem Wachstumsfaktor und/oder Cytokin und/oder Hormon durchgeführt wird.

6. Verfahren nach mindestens einem der vorherigen Ansprüche, wobei aus Säugetiergewebe isolierte Chondrocyten kultiviert werden.

7. Verfahren nach einem der vorherigen Ansprüche, wobei Chondrocyten aus Chondrocytenvorläuferzellen und/oder mesenchymalen Stammzellen und/oder aus embryonalen Stammzellen und/oder aus adulten Stammzellen kultiviert werden.

8. Verfahren nach Anspruch 6, wobei die Chondrocyten humanen Ursprungs sind.

9. Verfahren nach einem der vorherigen Ansprüche, wobei die Zellen, bevorzugt Chondrocyten, in Gewebekulturflaschen ausgesät werden und in einer Monolayerkultur mit mit FCS versetztem Medium kultiviert werden und die Konzentration von Magnesium anfänglich im Bereich von 11 bis 25 mMol liegt.

10. Verfahren nach einem der vorherigen Ansprüche, wobei bei Erhöhung der Magnesiumkonzentration die Zellen in Alginat eingebettet sind und in einem mit Serum aus einem Säugetier versetzten Medium kultiviert werden, die Konzentration an Magnesium auf einem Bereich von 21 bis 65 mMol erhöht wird.

11. Verfahren nach Anspruch 10, wobei die Kultivierung unter einem Sauerstoffpartialdruck von 8 % durchgeführt wird.

12. Verfahren zur Herstellung von Knorpelgewebe, umfassend das Verfahren gemäß einem der Ansprüche 1 bis 11.

13. Verwendung von gemäß einem der Ansprüche 1 bis 11 erhaltenen Chondronen zur Herstellung von Knorpelgewebe.

14. Knorpelgewebe erhalten gemäß einem Verfahren nach Anspruch 12.

15. Die Verwendung von Mg und Mg-Derivaten und Chondronen durch ein Verfahren gemäß einem der Ansprüche 1 bis 11 in-vitro-generiert zur Herstellung eines Medikaments zur Behandlung von Patienten, die an Knorpelerkrankungen, -störungen oder Beschädigungen aufgrund eines chirurgischen Eingriffs, Degeneration oder als Konsequenz von anderen Erkrankungsarten leiden, wobei die extrazelluläre Magnesiumkonzentration auf ein unphysiologisch hohes extrazelluläres Niveau erhöht wird und diese unphysiologisch hohe extrazelluläre Magnesiumkonzentration mindestens einmal während der Behandlung weiter erhöht wird.

16. Verwendung nach Anspruch 15, wobei das Magnesium eine Lösung von Magnesiumsulfat oder Magnesiumchlorid ist.

17. Verwendung nach einem der Ansprüche 15 oder 16, wobei das Medikament weiterhin mindestens einen Wachstumsfaktor und/oder Cytokin und/oder Hormon enthält.

18. Verwendung nach einem der Ansprüche 15 bis 17, wobei das Magnesium zur Verabreichung in Form eines Substrates oder eines Mittels angepasst ist.

19. Verwendung nach einem der Ansprüche15 bis 18, wobei das Medikament zur Verabreichung in dem oder in die Nähe des zu behandelnden Knorpels angepasst ist.

20. Verwendung nach einem der Ansprüche 15 bis 19, wobei die Knorpelerkrankung eine ist aus chondralen oder osteochondralen Defekten oder Beschädigungen, die Ruptur oder Degeneration von Meniskus oder Diskus, bei degenerativer, autoimmuner oder imflammatorischer Erkrankung oder Trauma, die zu Hyalin-, elastischen und/oder fibroelastischen Knorpelschädigungen führen.

21. Verwendung von Chondron erhaltenen gemäß einem der Ansprüche 1 bis 11 oder von Knorpelgewebe erhaltenen gemäß Anspruch 14 zur Herstellung eines Medikaments zur Behandlung von Patienten, die an Knorpelerkrankungen, Störungen oder Beschädigungen aufgrund von chirurgischen Eingriffen, Trauma, Degeneration oder als Konsequenz von anderen Erkrankungsarten leiden, wobei die extrazelluläre Magnesiumkonzentration auf ein unphysiologisch hohes extrazelluläres Niveau erhöht wird und diese unphysiologisch hohe extrazelluläre Magnesiumkonzentration ist mindestens einmal während der Behandlung weiter erhöht.

## Revendications

1. Procéde pour la génération in vitro de chondrons comprenant l'étape de:
culture de cellules dans un milieu comportant une concentration de magnesium (Mg) extracellulaire élevée, non physiologique, **caractérisée en ce que** la concentration de magnesium extra cellules.

2. Procédé selon la revendiaction 1, dans lequel ledit magnesium est une solution des sulphate de magnesium ou de chlorure de magnesium.

3. Procédé selon l'une quelconque des revendications 1 ou 2, dans lequel lesdites concentrations extra cellulaires de ladite solution de magnesium sont dans une gamme d'environ 12 mMol à environ 65 mMol.

4. Procédé selon l'une quelconque des revendications précédentes, dans lequel la culture des cellules est en outré affectée en la presence des serum foetal des veau (FCS) ou des sérum de mammifère.

5. Procédé selon l'une quelconque des revendications précédents, dans lequel la culture de cellules est en outré affectée en la présence d'au moins un facteur des croissance et/ou cytokine et/ou hormone.

6. Procédé selon l'une quelconque des revendications précédents, dans lequel des chondrocytes isolés du tissue d'un mammifère sont cultivés.

7. Procédé selon l'une quelconque des revendiations précédents, dans lequel des chondrocytes différenciés provenant des cellules précurseurs de chondrocytes et/ou de cellules souches mésenchymateuses et/ou des cellules souches embryonnaires et/ou de cellules souches adultes son cultivés.

8. Procédé selon la revendication 6, dans lequel les chondrocytes sont d'origine humaine.

9. Procédé selon l'une quelconque des revendications precedents, dans lequel les cellules, de préférence des chondrocytes, sont semées dans des flacons de culture tissulaire et sont sultivées en culture monocouche avec un milieu additionné des FCS et la concentration des magnesium est initialement dans la gamme de 11 à 25 mMol.

10. Procédé selon l'une quelconque des revendications précédentes, dans lequel lorsque la concentration de magnesium est augmentée les cellules sont incluses dans de l'alginate et cultivées dans un milieu additionné des serum dudit mammifère, la concentration de magnesium est augmentée jusqu'à une gamme de 21 à 65 mMol.

11. Procédé selon la revendication 10 dans lequel la culture est effectuée sous une pression partielle d'oxygène de 8%.

12. Procédé pour la preparation de tissue cartilagineux comprenant le procédé selon l'une quelconque des revendications 1 à 11.

13. Utilisation des chondrons obtenus selon l'une quelconque des revendications 1 à 11 pour la preparation de tissue cartilagineux.

14. Tissu cartilagineux obtenu selon le procédé de la revendication 12.

15. Utilisation de Mg et de dérivés de Mg et de chondons générés in vitro par un procédé selon l'une quelconque des revendications 1 à 11 pour la preparation d'un medicament pour traiter des patients souffrant des cartilages par maladies, désordres ou dommage dus à la chirurgie, traumatismes, dégénérescence ou en consequence d'autres types de maladies, par laquelle la concentration de magnesium extracellulaire est augmentée à un niveau extracellulaire élevé, non physioligique, et ladite concentration extracellulaire élevée, non physiologique de Mg est encore augmentée au moins une fois Durant le traitement.

16. Utilisation selon la revendication 16, dans laquelle ledit magnesium est une solution de sulphate de magnesium ou de chlorure de magnesium.

17. Utilisation selon l'une quelconque des revendications 15 ou 16 laquelle le medicament contient en plus au moins un facteur de croissance et/ou cytokine cite et/ou hormone.

18. Utilisation selon l'une quelconque des revendications 15 à 17 dans laquelle le magnesium est adapté à l'administré sous la forme d'un substrat ou d'un agent.

19. Utilisation selon l'une quelconque des revendications 15 à 18, dans laquelle le medicament est adapté à l'administré dans ou à proximité du cartilage à traiter.

20. L'utilisation selon l'une quelconque des revendications 15 à 19, dans laquelle la maladie des cartilages est l'un quelconque des défauts ou dommages chonraux ou ostéochondraux, la rupture ou la dégénérescence de ménisques ou de disques, dans une maladie degenerative, autoimmune, ou inflammatoire ou un traumatisme menant à un dommage du cartilage hyaline, èlastique et/ou fibroélastique.

21. Utilisation de chondrons obtenus selon l'une quelconque des revendications 1 à 11 ou de tissue cartilagineux selon la revendication 14 pour la preparation d'un medicament pou traite des patients souffrant des cartilages par maladies, désordres ou damages dus à la chirurgie, traumatismes, dégénérescence ou en conséquence d'autres types de maladies par laquelle la concentration de magnesium extracellulaire est augmentée à un niveau extracellulaire èlevé, non physilogique, et ladite concentration de Mg extracellulaire élevée, non physiologique, est encore augmentée au moins une fois Durant le traitement.
